(19) Europäisches Patentamt
European Patent Office
Office européen des brevets

(11) Veröffentlichungsnummer: **0 098 466**
**B1**

(12) # EUROPÄISCHE PATENTSCHRIFT

(45) Veröffentlichungstag der Patentschrift: **29.01.86**

(51) Int. Cl.⁴: **A 61 F 2/38**

(21) Anmeldenummer: **83106128.8**

(22) Anmeldetag: **23.06.83**

(54) **Implantierbares Ellbogengelenk.**

(30) Priorität: **26.06.82 DE 3223925**

(43) Veröffentlichungstag der Anmeldung: **18.01.84 Patentblatt 84/3**

(45) Bekanntmachung des Hinweises auf die Patenterteilung: ***29.01.86* Patentblatt 86/5**

(84) Benannte Vertragsstaaten: **AT BE CH DE FR GB IT LI LU NL SE**

(56) Entgegenhaltungen:
**EP-A-0 006 314**
**DE-A-2 823 406**
**US-A-3 547 115**

**BIOMEDIZINISCHE TECHNIK, Band 25, Nr. 4, April 1980, *Seiten 74-80*, Berlin, DE. R. Ebert et al.: "Keramische Werkstoffe in der Ersatzteilchirurgie"**

(73) Patentinhaber: **Feldmühle Aktiengesellschaft, Fritz-Vomfelde-Platz 4, D-4000 Düsseldorf 11 (DE)**

(72) Erfinder: **Dörre, Erhard, Dr., Talweg 14, D-7310 Plochingen (DE)**
Erfinder: **Prüssner, Peter, Ing. grad, Waldstrasse 38, D-6057 Dietzenbach (DE)**
Erfinder: **Zichner, Ludwig, Dr., Am Mühlberg 8, D-6000 Frankfurt/M. (DE)**

(74) Vertreter: **Uhlmann, Hans, Dr. rer.nat., Dipl.-Chem., Gladbacher Strasse 189, D-4060 Viersen 1 (DE)**

**EP 0 098 466 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft ein implantierbares Ellbogengelenk zur Verbindung von Oberarm und Unterarm, bestehend aus einer Hülse mit C-förmigem Querschnitt zur Umfassung der Rolle des Oberarmknochens um mehr als 180 Grad und als Gegenlager zur Abstützung der Speiche sowie einem in die Elle verankerbaren Verbindungsstück.

Ellbogengelenke, die das gesamte Gelenk ersetzen, also eine Resektion des Gesamtgelenkbereiches erfordern, sind beispielsweise aus der DE-B Nr. 2007214 und der DE-A Nr. 2351912 bekannt. Man ist jedoch bestrebt, einen solchen Austausch des gesamten Ellbogengelenkes nach Möglichkeit zu vermeiden und die Knochen des Ellbogengelenkes so weit wie irgend möglich zu erhalten, um für den Fall einer weiteren Knochenrückbildung oder Erkrankung Rückzugsmöglichkeiten zu haben.

Es ist deshalb bereits mit der DE-A Nr. 2359627 vorgeschlagen worden, die Rolle (Trochlea) des Oberarmknochens so zu bearbeiten, dass mit Acrylharzzement eine Kappe auf ihr befestigt werden kann. Diese Kappe umgreift die resezierte Rolle und weist als äusseren Mantel sowohl einen sphärischen als auch einen zylindrischen Bereich auf. Der spärische Bereich steht dabei mit einer Kugelpfanne mit Dornansatz in Verbindung, die in die Speiche eingesetzt wird, der das Köpfchen reseziert wurde, während in die Elle eine zylindrische Pfanne eingekittet wird. Beim Aushärten von Knochenzementen entstehen jedoch so hohe Temperaturen, dass eine Schädigung des Knochengewebes auftreten kann. Man versucht daher nach Möglichkeit eine Verankerung mit Knochenzement zu vermeiden.

Die DE-A Nr. 2823406 beschreibt ein künstliches Ellbogengelenk, bei dem die Rolle von einer C-förmigen Oberarmkomponente aus rostfreiem Stahl umgriffen wird, die in ihrem zylindrischen Innenbereich Aussparungen oder Aufrauhungen besitzt, durch die sie mittels Knochenzement mit der resezierten Rolle verbunden werden kann. Die Oberarmkomponente weist dabei drei sphärische Abschnitte auf, die im wesentlichen der Ausbildung der Rolle entsprechen. Das Rollenende wird ebenfalls reseziert und mit einer sattelförmigen Gelenkfläche versehen, die mit Kunststoff ausgekleidet ist. Diese Gelenkfläche gleitet auf der Oberarmkomponente.

Nach dem Vorschlag dieses Standes der Technik ist es immer erforderlich, eine Gelenkkomponente, nämlich die Rolle, mit Knochenzement am Oberarmknochen zu befestigen.

Aufgabe der vorliegenden Erfindung ist es daher, ein implantierbares Ellbogengelenk zu schaffen, das diese Nachteile nicht aufweist und unter Beibehaltung der beiden physiologischen Bewegungsmöglichkeiten des Ellbogengelenks, nämlich Beugen und Drehen, auch bei geschädigten Bändern eine sichere Verbindung zwischen Oberarm und Unterarm gewährleistet, möglichst wenig Knochenresektion erfordert, um die Möglichkeit einer Nachoperation offenzulassen, den Einsatz neuer, hochverschleissfester Werkstoffe ermöglicht, die nicht die Nachteile bisheriger, künstlicher Ellbogengelenke aus oder unter Mitverwendung von Metall als Gleitpartner aufweisen, wie Abrieb von Metallstaub, der toxisch wirkt und zu einer Lockerung der Verbindung mit dem Knochen führt, durch die konstruktive Gestaltung Erleichterungen für den Operateur schafft.

Dieses komplexe Aufgabenspektrum lässt sich nicht erfüllen durch eine blosse Nachbildung des natürlichen Ellbogengelenks, sondern erfordert von der Konstruktion und vom Material her gänzlich neue Wege.

Diese Aufgabe wird gelöst durch eine implantierbares Ellbogengelenk zur Verbindung von Oberarm und Unterarm, bestehend aus einer Hülse mit C-förmigem Querschnitt zur Umfassung der Rolle des Oberarmknochens um mehr als 180 Grad und als Gegenlager zur Abstützung der Speiche, sowie einem in die Elle verankerbaren Verbindungsstück, bei dem erfindungsgemäss neu folgende Merkmale kombiniert sind:

Die Hülse weist einen sich radial erstreckenden Schlitz auf;

die Innenwandung der Hülse bildet zumindest im Bereich des Schlitzes einen Innenzylinder;

in der Hülse ist im Bereich des Schlitzes ein zylindrisches Gleitstück gelagert;

ein zur Verankerung in der Elle bestimmter Schaft erstreckt sich mit seinem freien Ansatz durch den Schlitz hindurch und greift in das Gleitstück ein.

Kern der Erfindung sind das in der Hülse gelagerte Gleitstück und der Schlitz in der Hülse. Dadurch werden in hervorragender Weise nachfolgende Funktionen erfüllt und Vorteile erzielt:

1. Das Gleitstück stellt durch seine Lagerung in der Hülse und in einer entsprechenden Ausnehmung in der Rolle, sowie über den durch den Schlitz hindurchgreifenden Ansatz des Schaftes, die Verbindung zwischen Oberarm und Unterarm her.

2. Das Gleitstück nimmt die Scharnierbewegung auf mit dem ganz besonderen Vorteil, dass die gleitenden und auf Verschleiss beanspruchten Teile innerhalb der Hülse liegen – im Gegensatz zum natürlichen Gelenk, bei dem diese Scharnierbewegung über die Aussenfläche der Rolle mit der entsprechenden Gegenfläche am Ende der Elle (incisura trochlearis) abläuft.

Ein weiterer Vorteil dieses konstruktiven Gestaltungsmerkmals liegt darin, dass bei krankhaftem Verschleiss der incisura trochlearis kein Ersatz erforderlich ist, da die Funktion der Scharnierbewegung von Hülse und Gleitstück übernommen wird.

3. Diese konstruktive Ausgestaltung lässt sich zementfrei implantieren, weil durch das Zusammenwirken von Hülse und Gleitstück eine einfache und sichere Verbindung geschaffen ist.

4. Diese konstruktive Ausgestaltung ist operativ einfach und sicher und erhält soviel wie möglich an natürlicher Substanz. Es wird lediglich die Rolle entsprechend der Form und den Abmessungen des Innenzylinders der Hülse zugearbeitet, ein Zwischenstück zur Einfügung des Gleitstückes entfernt, das Gleitstück eingefügt und dann die Hülse auf die bearbeitete Rolle und das Gleitstück aufgeschoben.

Die Drehbewegung des Ellbogengelenkes dagegen wird in der natürlichen Art und Weise aufrechterhalten, indem der Endteil der Hülse, der zu diesem Zweck ballig ausgeführt ist, die Funktion dieses Teiles der Rolle, des capitulum humeri übernimmt und im gleitenden Eingriff steht mit dem Köpfchen der Speiche, dem caput radii. Es liegt natürlich im Rahmen der Erfindung, auch das Köpfchen durch ein entsprechendes Implantat zu ersetzen, sollte dies krankhaft verändert sein.

5. Ein ganz wesentlicher weiterer Vorteil des erfindungsgemässen implantierbaren Ellbogengelenks liegt darin, dass die Konstruktion ausgesprochen materialgerecht ist, insbesondere bei alleiniger oder Mitverwendung von Implantatteilen aus gesinterten Hartstoffen. Gesinterte Hartstoffe, insbesondere gesintertes Aluminiumoxid zeichnen sich durch grosse Verschleissfestigkeit und ausgezeichnete Reibeigenschaft aus. Sie sind aber von so ausserordentlicher Härte, die fast die des Diamanten erreicht, dass ihre Bearbeitung sehr aufwendig ist und eine Bearbeitung entsprechend den komplizierten Formen des menschlichen Knochens einen hohen Aufwand bedeuten würde. Deshalb ist man bei Verwendung dieser Werkstoffe bestrebt, gleichmässige, einfache geometrische Formen zu haben, die mit üblichen Werkzeugen geformt und in üblicher Weise geschliffen und poliert werden können. Diesem Erfordernis kommt die Ausbildung der Innenfläche der Hülse als Innenzylinder und des Gleitstücks als einfache Zylinder ausgesprochen entgegen.

Wenn auch das implantierbare Ellbogengelenk aus den bisher üblichen Materialien, wie Metall oder Kunststoff, bestehen kann, so ist es jedoch wie bereits erwähnt, besonders vorteilhaft, das Gleitstück, die Hülse oder beide aus gesinterten Hartstoffen herzustellen. Dabei hat sich gesintertes Aluminiumoxid seiner grossen Härte, Verschleissfestigkeit und chemischen Inertheit wegen besonders bewährt. Bevorzugt wird ein Aluminiumoxid mit einem $Al_2O_3$-Gehalt von mindestens 99,5 Gew.%, einer Dichte von mehr als 3,9 und einer mittleren Korngrösse $< 10$ µm. Ausser grosser mechanischer Festigkeit und Verschleissfestigkeit zeichnet sich ein solches Material dadurch aus, dass es gut polierfähig ist. Das ist deshalb besonders von Bedeutung, weil von den miteinander gleitend im Eingriff stehenden zylindrischen Flächen, zumindest die des Hartstoffkörpers, vorzugsweise auf eine mittlere Oberflächenrauhigkeit von $< 0,1$ µm poliert werden.

Vom Verschleissverhalten und vom Reibungsverhalten her stellt ein implantierbares Ellbogengelenk, bei dem sowohl die Hülse als auch das Gleitstück aus gesintertem Aluminiumoxid bestehen, die optimale Ausführungsform dar, insbesondere deshalb, weil beim Vorhandensein von Körperflüssigkeit, wie das beim Ellbogengelenk der Fall ist, Aluminiumoxid auf Aluminiumoxid ein ausgezeichnetes Reibverhalten zeigt und praktisch kein Abrieb auftritt, der als solcher noch dazu körperverträglich wäre und nicht die Nachteile hätte wie beim bisher üblichen Metall.

Eine andere zweckmässige Ausführungsform des erfindungsgemässen implantierbaren Ellbogengelenks besteht darin, dass das Gleitstück aus einem Polyäthylen hoher Dichte hergestellt ist, die Hülse aus gesintertem Aluminiumoxid. Die Materialpaarung Polyäthylen gegen gesintertes Aluminiumoxid zeichnet sich ebenfalls durch ein ausgesprochen gutes Reibverhalten und geringen Verschleiss aus.

Als Material für den Schaft, d.h. den Teil, der in der Elle verankert wird und mit seinem freien Ansatz durch den Schlitz hindurch in das Gleitstück eingreift, wird bevorzugt Metall gewählt, mit Rücksicht auf seine grössere Biegebeanspruchbarkeit. Besonders bewährt haben sich Titanlegierungen und Legierungen aus Chrom, Nickel, Kobalt und Molybdän. Denkbar sind auch Kohlefaserwerkstoffe und Polyäthylen.

Besondere Bedeutung kommt noch der Verbindung des Schaftes mit dem Gleitstück zu. Hier hat sich besonders bewährt, dass der Schaft an seinem freien Ansatz einen Konus aufweist, der in die konische Bohrung des Gleitstückes eingreift und selbsthemmend eine sichere Verbindung herstellt. Bevorzugt hat der Konus ein Verjüngungsverhältnis zwischen 1:10 und 1:20. Insbesondere bei Verwendung eines Gleitstückes aus gesintertem Aluminiumoxid weist der Mutterkonus im Gleitstück bevorzugt eine grössere Rauhigkeit auf, zweckmässig einen arithmetischen Mittenrauhwert Ra zwischen 0,5 und 3 µm, so dass der Mutterkonus durch seine Härte und die Rauhigkeit auf den Vaterkonus aus Metall verformend einwirkt. Das ist deshalb von besonderem Vorteil, weil gesintertes Aluminiumoxid an sich gegenüber Zugbeanspruchung empfindlich ist und durch diese Verformungskräfte, die Sprengkräfte auf das Gleitstück aufgefangen werden.

Weitere Vorteile und zweckmässige Ausgestaltungen des Erfindungsgegenstandes ergeben sich aus den nachfolgend beschriebenen Zeichnungen, ohne dass der Anmeldegegenstand auf diese ganz konkrete Ausführungsform beschränkt ist.

Fig. 1 zeigt im Überblick die Einzelteile des implantierbaren Ellbogengelenkes und ihre Zuordnung zu Oberarmknochen, Elle und Speiche,

Fig. 2 zeigt das in den Ober- und Unterarm implantierte Ellbogengelenk im Schnitt von der Seite her gesehen,

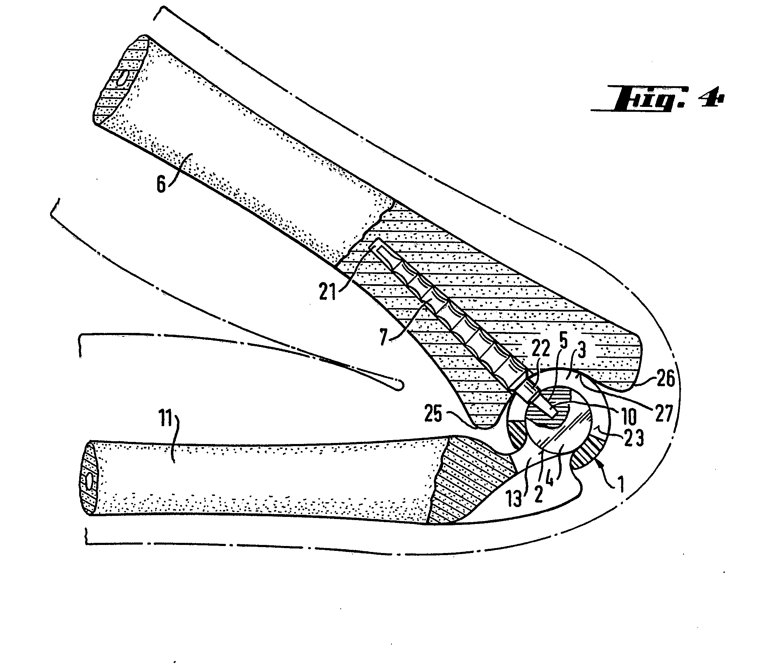

Fig. 3 die Ansicht von vorn,

Fig. 4 zeigt die Seitenansicht im Schnitt in Beugestellung des Armes.

Das implantierbare Ellbogengelenk besteht aus Hülse 1, Gleitstück 4 und Schaft 7.

Die Hülse 1, die über ihre gesamte Länge annähernd gleiche Wandstärken aufweist, ist in ihrem Umfang nicht gänzlich geschlossen, sondern der Hülsenmantel umfasst nur ca. 270 Grad, damit er noch auf die Rolle 17 des Oberarmknochens 11 aufgeschoben werden kann. Die Hülse 1 ist an dem Teil, der zum gleitenden Eingriff mit dem Köpfchen 24 der Speiche 16 bestimmt ist, durch einen sphärischen Bereich 8 ballig ausgebildet, der vorzugsweise poliert ist, um die Reibung gegenüber dem Köpfchen 24 gering zu halten. In Form und Abmessung entspricht dieser sphärische Bereich 8 weitgehend dem capitulum humeri 19 von der Resektion. Im hier gezeigten Beispiel ist das Köpfchen 24 noch in seiner natürlichen Form erhalten, also nicht durch Krankheit oder Unfall zerstört. Sollte das der Fall oder erkennbar sein, dass sich die Krankheit in absehbarer Zeit auch auf das Köpfchen 24 ausdehnt, wird das Köpfchen 24 zweckmässig ebenfalls durch ein entsprechendes Implantat ersetzt.

Der an den sphärischen Bereich 8 anschliessende Teil der Hülse 1 ist im wesentlichen zylindrisch ausgeführt und weist einen Schlitz 3 auf, der bevorzugt ca. 120 Grad des Gesamtumfanges der Hülse umfasst und durch den der Ansatz 22 des Schaftes 7 hindurchgreift. Im Durchmesser ist der Ansatz 22 um ein Geringes kleiner als die Breite des Schlitzes 3. Der Schaft 7, der rippenförmige Verstärkung zur besseren Befestigung aufweist, dient zur Verankerung in einer in die Elle 6 eingebrachten Bohrung 21. Der Ansatz 22 gleitet bei Bewegung des Armes an den Seitenflächen 23 des Schlitzes 3 entlang. Um auch hier geringe Reibung und keinen Abrieb zu haben, sind die Seitenflächen 23 und die Oberfläche des Ansatzstückes 22 zweckmässig poliert. Der Schaft 7 endet – anschliessend an den Ansatz 22 – im Konus 10, mit einem Verjüngungsverhältnis zwischen 1:10 und 1:20.

Das Gleitstück 4 weist Zylinderform auf, ist in seiner Länge mindestens doppelt so gross gehalten wie die Breite des Schlitzes 3 und ist beim implantierten Ellbogengelenk in der Hülse 1 gelagert und steht mit dieser gleitend im Eingriff. Zu diesem Zweck entspricht der Durchmesser des Gleitstücks 4 im wesentlichen der lichten Weite der Hülse 1, so dass das Gleitstück 4 mit seiner polierten Mantelfläche 2 mit dem Innenzylinder 14 von 1, der ebenfalls poliert ist, gleitend im Eingriff steht. Das Gleitstück 4 ist in seiner Länge deshalb mindestens doppelt so lang wie der Schlitz 3 breit ist, damit eine sichere Führung gewährleistet ist.

Bei der Implantation wird aus der Rolle 17 mittels eines Scheibenfräsers eine Aussparung 13 herausgeschnitten, die in ihrer Breite der Höhe des Gleitstückes 4 entspricht. Da das Gleitstück 4 mit seinen Stirnflächen 15 mit den Seitenflächen der Aussparung 13 gleitend und reibend im Eingriff steht, sind bevorzugt auch die Stirnflächen 15 poliert. Die Elle 6 ist im Bereich der ursprünglichen Gelenkfläche, nämlich des Rabenschnabelfortsatzes 25 (Coronoideus) und des Ellenhakens 26 (Olecranon) in einer Weise reseziert, die der zylindrischen Aussenfläche der Hülse 1 in diesem Bereich entspricht, so dass sich eine bogenförmige Ausnehmung 27 ergibt.

Die Implantation erfolgt ungefähr wie folgt:

Zunächst wird die Rolle 17 zylinderförmig zugearbeitet. Dabei ist es nicht erforderlich, die gesamte Rolle zuzuarbeiten, sondern es kann, wie das in Fig. 1 dargestellt ist, beispielsweise ein unbearbeiteter Bereich 18 stehen bleiben, der bei der Rolle 17 den Übergang zwischen dem bearbeiteten capitulum humeri und den zugearbeiteten Knorren 12 (Condylen) bildet. In die Rolle 17 ist mittels eines Scheibenfräsers eine Aussparung 13 eingebracht, deren Breite der Höhe des Gleitstückes 4 entspricht. Wie mit Pfeil 20 angedeutet, wird in diese Aussparung 13 das Gleitstück 4 eingesetzt, und zwar in der Weise, dass die konische Bohrung 5 in entgegengesetzter Richtung zum Oberarmknochen 11 weist.

Über die bearbeitete Rolle 17 und das in die Aussparung 13 eingefügte Gleitstück 4 wird von der Seite her, wie ebenfalls durch Pfeile angedeutet, die Hülse 1 aufgeschoben. Die Verbindung zur Elle 6, in die der Schaft 7 eingelassen ist, erfolgt dann in einfacher Weise durch Hindurchführen des Ansatzes 22 durch den Schlitz 3 und Einstekken des Konus 10 in die konische Bohrung 5.

## Patentansprüche

1. Implantierbares Ellbogengelenk zur Verbindung von Oberarm und Unterarm, bestehend aus einer Hülse (1) mit C-förmigem Querschnitt zur Umfassung der Rolle (17) des Oberarmknochens (11) um mehr als 180 Grad und als Gegenlager zur Abstützung der Speiche (16) sowie einem in die Elle (6) verankerbaren Verbindungsstück (7), gekennzeichnet durch die Kombination nachfolgender Merkmale:

Die Hülse (1) weist einen sich radial erstreckenden Schlitz (3) auf;

die Innenwandung der Hülse (1) bildet zumindest im Bereich des Schlitzes (3) einen Innenzylinder (14);

in der Hülse (1) ist im Bereich des Schlitzes (3) ein zylindrisches Gleitstück (4) gelagert;

ein zur Verankerung in der Elle (6) bestimmter Schaft (7) erstreckt sich mit seinem freien Ansatz (22) durch den Schlitz (3) hindurch und greift in das Gleitstück (4) ein.

2. Implantierbares Ellbogengelenk nach Anspruch 1, dadurch gekennzeichnet, dass das zylindrische Gleitstück (4) mindestens doppelt so lang ist, wie der Schlitz (3) breit.

3. Implantierbares Ellbogengelenk nach einem der Ansprüche 1 und 2, dadurch gekennzeichnet, dass die Hülse (1) in den an den Schlitz (3) angrenzenden Bereichen des Innenzylinders (14) poliert ist.

4. Implantierbares Ellbogengelenk nach einem der Ansprüche 1 bis 3, dadurch gekennzeichnet, dass die Mantelfläche des Gleitstückes (4) poliert ist.

5. Implantierbares Ellbogengelenk nach einem der Ansprüche 1 bis 4, dadurch gekennzeichnet, dass die Stirnflächen (15) des Gleitstückes (4) poliert sind.

6. Implantierbares Ellbogengelenk nach einem der Ansprüche 1 bis 5, dadurch gekennzeichnet, dass das Gleitstück (4) eine konische Bohrung (5) aufweist und der Schaft (7) mit einem Konus (10) zum Eingriff in die konische Bohrung (5) des Gleitstücks (4) versehen ist.

7. Implantierbares Ellbogengelenk nach einem der Ansprüche 1 bis 6, dadurch gekennzeichnet, dass die Hülse (1) und/oder Gleitstück (4) aus gesinterten Hartstoffen besteht.

9. Implantierbares Ellbogengelenk nach einem der Ansprüche 1 bis 7 und Anspruch 8, dadurch gekennzeichnet, dass der gesinterte Hartstoff Aluminiumoxid mit einem $Al_2O_3$-Gehalt von mindestens 99,5 Gew.%, einer Dichte von mehr als 3,9 und einer mittleren Korngrösse $< 10$ µm ist.

10. Implantierbares Ellbogengelenk nach einem der Ansprüche 1 bis 7 und einem der Ansprüche 8 und 9, dadurch gekennzeichnet, dass von den miteinander gleitend im Eingriff stehenden zylindrischen Flächen (Mantelfläche 2, Innenzylinder 14) zumindest die des Hartstoffkörpers auf eine mittlere Oberflächenrauhigkeit von $< 0,1$ µm poliert ist.

11. Implantierbares Ellbogengelenk nach einem der Ansprüche 1 bis 10, dadurch gekennzeichnet, dass die Hülse (1) aus gesintertem Aluminiumoxid, das Gleitstück (4) aus Polyäthylen hoher Dichte besteht.

## Claims

1. Implantable elbow joint for conecting the upper arm and the lower arm, comprising a sleeve (1) of C-shaped cross-section for encircling the trochlea (17) of the humerus (11) over more than 180 degrees and, as an abutment, for supporting the radius (16), and a connecting piece (7) that can be anchord in the ulna (6), characterized by the combination of the following features:

the sleeve (1) has a radially extending slot (3);

the inner wall of the sleeve (1) forms an inner cylinder (14) at least in the region of the slot (3);

a cylindrical slide (4) is mounted in the sleeve (1) in the region of the slot(3);

the free and portion (22) of a shaft (7) intended for anchoring in the ulna (6) extends through the slot (3) and engages the slide (4).

2. Implantable elbow joint according to Claim 1, characterized in that the cylindrical slide (4) is at least twice as long as the slot (3) is wide.

3. Implantable elbow joint according to Claim 1 or 2, characterized in that the sleeve (1) is polished in the regions of the inner cylinder (14) that border on the slot (3).

4. Implantable elbow joint according to any one of Claims 1 to 3, characterized in that the surface (2) of the slide (4) is polished.

5. Implantable elbow joint according to any one of Claims 1 to 4, characterized in that the end faces (15) of the slide (4) are polished.

6. Implantable elbow joint according to any one of Claims 1 to 5, characterized in that the slide (4) has a conical bore (5) and the shaft (7) is provided with a cone (10) for engaging the conical bore (5) in the slide (4).

7. Implantable elbow joint according to any one of Claims 1 to 6, characterized in that the sleeve (1) has approximately the same wall thicknesses over its entire length.

8. Implantable elbow joint according to any one of Claims 1 to 7, characterized in that the sleeve (1) and/or the slide (4) consist(s) of sintered mechanically resistant materials.

9. Implantable elbow joint according to any one of Claims 1 to 7 and Claim 8, characterized in that the sintered mechanically resistant material is aluminium oxide having an $Al_2O_3$-content of at least 99.5% by weight, a density of more than 3.9 and a mean particle size of $< 10$ µm.

10. Implantable elbow joint according to any one of Claims 1 to 7 and Claim 8 or 9, characterized in that of the cylindrical surfaces that are in sliding contact with one another (surface 2, inner cylinder 14), at least that of the component made of mechanically resistant material is polished to a mean surface roughness of $<0.1$ µm.

11. Implantable elbow joint according to any one of Claims 1 to 10, characterized in that the sleeve (1) consists of sintered aluminium oxide and the slide (4) consists of high-density polyethylene.

## Revendications

1. Articulation de coude, implantable, pour relier le bras à l'avant-bras, constituée par une douille (1) à section en forme de C pour entourer la trochlée (17) de l'humérus (11) sur plus de 180° et pour servir de contre-appui de soutien du radius (16), ainsi que par une pièce de liaison (7) capable d'être ancrée dans le cubitus, caractérisée par la combinaison des caractéristiques suivantes:

— la douille (1) présente une fente (3) s'étendant radialement;

— la paroi intérieure de la douille (1) forme, au moins dans la région de la fente (3), un cylindre interne (14);

— une pièce de glissement cylindrique (4) porte dans la douille (1), dans laquelle elle est logée, dans la région de la fente (3);

— une broche (7) destinée à l'ancrage dans le cubitus (6) s'étend, par son extrémité libre (22), au travers de la fente (3) qu'elle traverse et pénètre dans la pièce de glissement (4).

2. Articulation de coude, implantable, selon la revendication 1, caractérisée en ce que la pièce de glissement cylindrique (4) possède une longueur au moins double de la largeur de la fente (3).

3. Articulation de coude, implantable, selon l'une des revendications 1 et 2, caractérisée en ce que la douille (1) est polie dans les régions du cylindre interne (14) bordant la fente (3).

4. Articulation de coude, implantable, selon l'une des revendications 1 à 3, caractérisée en ce que la surface périphérique (2) de la pièce de glissement (4) est polie.

5. Articulation de coude, implantable, selon l'une des revendications 1 à 4, caractérisée en ce que les faces terminales (15) de la pièce de glissement (4) sont polies.

6. Articulation de coude, implantable, selon l'une des revendications 1 à 5, caractérisée en ce que la pièce de glissement (4) présente un trou conique (5) et la broche (7) est munie d'un cône (10) destiné à être mis en prise dans le trou conique (5) de la pièce de glissement (4).

7. Articulation de coude, implantable, selon l'une des revendications 1 à 6, caractérisée en ce que la douille (1) présente sensiblement la même épaisseur de paroi sur toute sa longueur.

8. Articulation de coude, implantable, selon l'une des revendications 1 à 7, caractérisée en ce que la douille (1) et/ou la pièce de glissement (4) est en matière dure frittée.

9. Articulation de coude, implantable, selon l'une des revendications 1 à 7 et la revendication 8, caractérisée en ce que la matière dure frittée est l'oxyde d'aluminium, la teneur de $Al_2O_3$ étant d'au moins 99,5% en poids, la densité étant supérieure à 3,9 et la grosseur de grain moyenne étant inférieure à 10 µm.

10. Articulation de coude, implantable, selon l'une des revendications 1 à 7 et l'une des revendications 8 et 9, caractérisée en ce que, parmi les surfaces cylindriques mutuellement conjuguées par glissement (surface périphérique 2, cylindre interne 14), au moins celle du corps en matière dure est polie à une rugosité moyenne inférieure à 0,1 µm.

11. Articulation de coude, implantable, selon l'une des revendications 1 à 10, caractérisée en ce que la douille (1) est en oxyde d'aluminium fritté, et la pièce de glissement (4) en polyéthylène à haute densité.

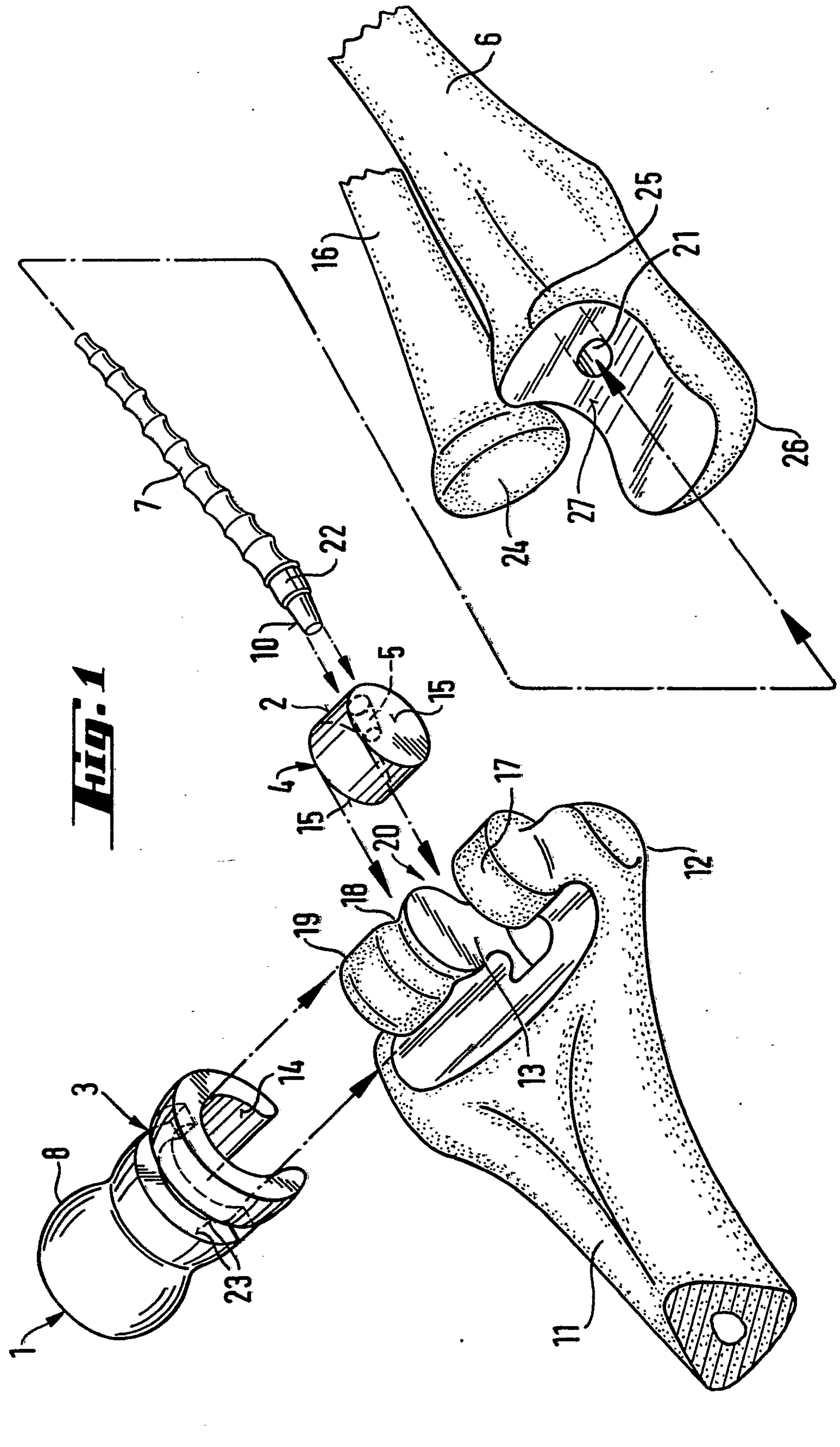

Fig. 1

Fig. 2

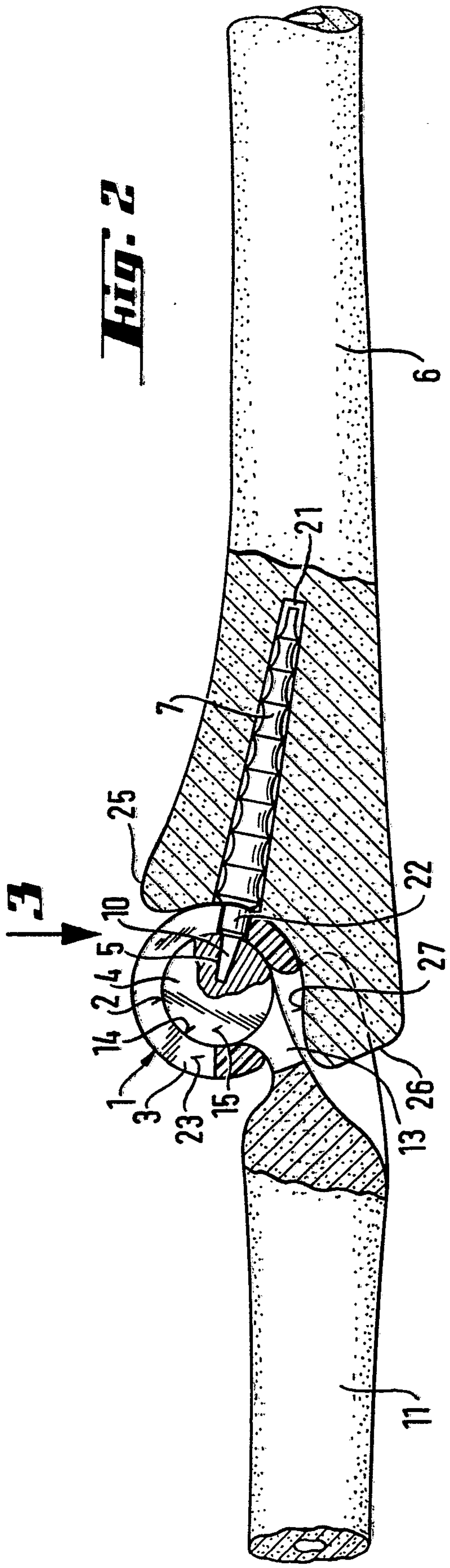
3
6
7
21
25
10
5
4
2
14
1
3
23
15
22
27
26
13
11

Fig. 3

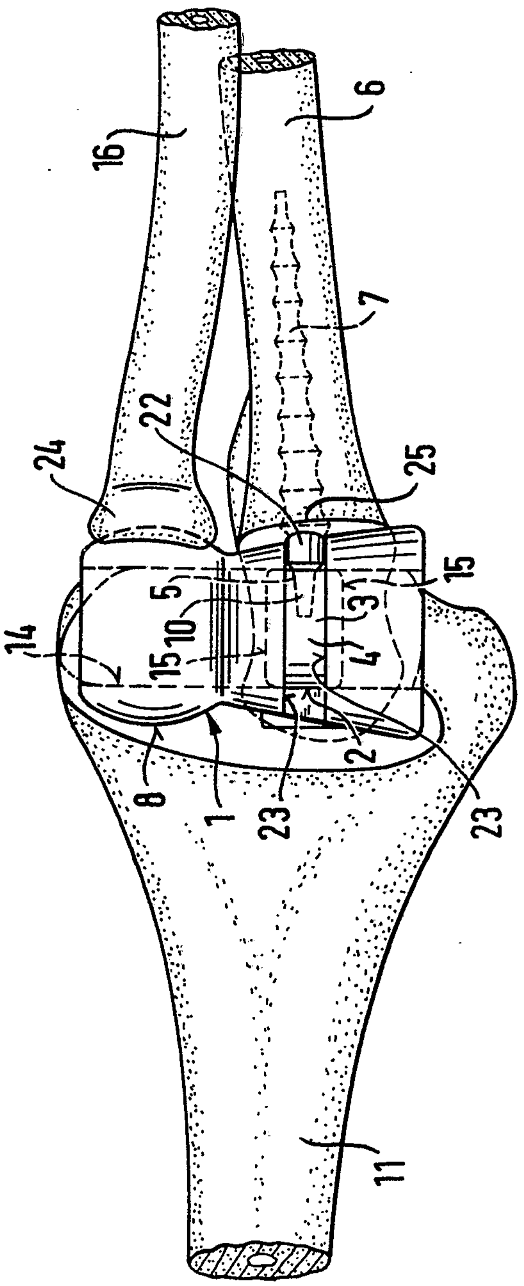
16
6
7
24
22
25
14
5
10
15
3
4
15
8
1
23
2
23
11

Fig. 4